# EUROPÄISCHE PATENTSCHRIFT

(11) **EP 0 683 628 B1**
(45) Veröffentlichungstag und Bekanntmachung des Hinweises auf die Patenterteilung: **20.05.1998**
(21) Anmeldenummer: 94907536.0
(22) Anmeldetag: 10.02.1994
(51) Int. Cl.: A01N 31/08

(54) **DESINFEKTIONSMITTEL MIT PARASITIZIDER WIRKSAMKEIT**
ANTI-PARASITIC DISINFECTANT
PRODUIT DESINFECTANT A ACTIVITE PARASITICIDE

(30) Priorität: 11.02.1993 DE 4304541; 24.02.1993 DE 4306336; 21.05.1993 DE 4317083
(43) Veröffentlichungstag der Anmeldung: 29.11.1995
(73) Patentinhaber: MENNO-CHEMIE-VERTRIEB GMBH, D-22850 Norderstedt (DE)
(72) Erfinder: NEVERMANN, Eugen, D-22397 Hamburg (DE)
(74) Vertreter: Vonnemann, Gerhard, Dr.-Ing.
(86) Internationale Anmeldenummer: EP9400382
(87) Internationale Veröffentlichungsnummer: WO9417661

(56) Entgegenhaltungen:
- EP-A- 0 339 448
- EP-A- 0 397 218

## Beschreibung

Die Erfindung betrifft ein Desinfektionsmittel zur Bekämpfung von Parasitosen und zur Abtötung von invasiven Dauerformen auf Basis eines Gemisches.

Der Befall durch Parasiten stellt in der Intensivtierhaltung unvermindert eine große Gefahr für den Züchterbetrieb dar. Trotz der Vielfalt der systemischen Behandlungsmöglichkeiten von Parasitosen bleibt der Einsatz von Medikamenten ohne nachhaltige Wirkung, wenn die Hygiene im Stall (Reinigung) nicht durch spezielle Desinfektionsmethoden ergänzt wird.

Haltung und Zuchterfolge bei Geflügel, Schweinen und Rindern werden dadurch beeinträchtigt, daß diese Tierarten von Kokzidien und Askariden befallen werden. Trotz Verfütterung von Wurm- und Kokzidienmitteln kommt es immer wieder zu Infektionen der Tiere, da diese mit ihrem Kot die sehr resistenten Dauerformen (Askarideneier und Kokzidienoozysten) der Schmarotzer ausscheiden, die invasionsfähig sind und durch die Nahrungsaufnahme erneut Eingang in den Tierkörper finden können.

Diese Parasiten schmarotzen im Darmtrakt und verursachen dabei umfangreiche Läsionen, die das Verkümmern oder den Tod der Tiere zur Folge haben.

Um dieser Gefahr zu begegnen, werden Desinfektionsmittel mit besonderen Wirkstoffzusätzen angewendet. Diese speziellen Zusätze sind meist chlorierte Lösungsmittel oder Schwefelkohlenstoff in emulgierter Form. Außerdem sind Schwefelkohlenstoff abspaltende Zweikomponentenpräparate in Gebrauch.

Aufgabe der genannten Lösungsmittel ist es, die sehr festen Membranen der parasitären Dauerformen zu durchdringen und so dem meist phenolischen Wirkstoff den Zutritt zum Inneren zu ermöglichen, wodurch es gelingt, die Parasiten irreversibel zu schädigen.

Der Anwendungsbereich der vorliegenden Erfindung liegt folglich im Bereich der Massentierhaltung, nicht aber in der Haushaltsreinigung oder medizinischen Anwendung. Deshalb berührt der Anmeldungsgegenstand keine bakterizide Wirksamkeit; die parasitizide Wirksamkeit ist Gegenstand der Erfindung. Die ebenfalls vorhandene bakterizide Wirksamkeit der Wirkstoffkombination ist ein unvermeidlicher Nebeneffekt.

Askariden gehören zu der Gattung der Spulwürmer, die Kokzidien hingegen zu den Telosporidien, parasitischen Protozoen (Urtierchen mit beweglichem Anfangsstadium). Sowohl Askariden als auch Kokzidien sind Kleinstlebewesen, die zoologisch aufgrund ihrer Größe, Morphologie und Lebensweise sowie ihres Fortpflanzungsmechanismus keinerlei Verwandtschaft mit Mikroben und Bakterien aufweisen. Askarideneier, z.B. der Art Askaris suum, weisen eine aus drei Schichten bestehenden Eihülle auf. Insbesondere die mittlere Schicht aus chitinähnlichen Substanzen und die an ihr innen anliegende Vitellinmembran sind nur schwer zu durchdringen. Die bisher üblichen Mittel gegen Askarideneier enthalten daher organische Lösemittel, chlorierte Kohlenwasserstoffe wie Tetrachlorkohlenstoff und Tetrachlorethylen oder Schwefelkohlenstoff, damit die phenolischen Wirkstoffe durch die Eihülle in das Innere gelangen können.

Derartige Desinfektionsmittel werden z.B. unter dem Namen Lomasept®L20 angeboten. Dieses Mittel enthält 36 % Trichlormethan, 24 % Schwefelkohlenstoff und 6,6 % 4-Chlor-3-methylphenol. Als Beispiel für ein schwefelkohlenstofffreies Desinfektionsmittel kann P3-incicoc® angeführt werden. Hier kommt neben 4-Chlor-3-ethyl-phenol Perchlorethylen im Gemisch mit Isopropanol zur Anwendung.

Besonders nachteilig an dieser dem Stand der Technik entsprechenden Methode ist, daß die zur Wirksamkeit der Desinfektionspräparate notwendigen Lösungsmittel aus ökologischer und toxikologischer Sicht als sehr bedenklich eingestuft werden. So sind in den haudelsüblichen Desinfektionspräparaten mit nachgewiesener parasitizider Wirksamkeit zwischen 5 - 20% Schwefelkohlenstoff oder zwischen 10 - 50% chlorierte Kohlenwasserstoffe (Perchlorethylen, Chloroform) enthalten.

Schwefelkohlenstoff ist sehr leicht entzündlich, explosionsgefährlich und giftig. Halogenierte Lösungsmittel gelten allgemein als besonders langlebige Umweltgifte.

Die EP 0 339 448 beschreibt ein parasitenabtötendes Desinfektionsmittel zur Bekämpfung und Abtötung von parasitären, invasionsfähigen Dauerformen. Das Mittel ist eine wässrige Desinfektionsmittelzusammensetzung auf der Basis von Gemischen aus Desinfektionswirkstoffen und oberflächenaktiven Wirkstoffen, die
a) mindestens einen Desinfektionswirkstoff aus der aus
   aliphatischen Aldehyden mit 1 bis 5 C-Atomen,
   aliphatischen Alkoholen mit 1 bis 5 C-Atomen,
   unsubstituierten und substituierten Phenolen,
   Aktivchlor-, Aktivsauerstoff- und
   Aktivjodverbindungen bestehenden Gruppe und
b) mindestens ein Sulfattensid der allgemeinen Formel (I)

   R¹ - O - (CₙH₂ₙO)ₘ -SO₃ M (I)

   in der R¹ für einen geradkettigen oder verzweigten,
   Alkyl- oder Alkenylrest mit 8 bis 18 C-Atomen, M für Wasserstoff, Lithium, Natrium, Kalium,
   C₁₋₄-Alkylammonium oder C₁₋₄-Alkanolammonium, n für 2 oder 3 und m für eine Zahl von 0 bis 15 stehen,
   enthalten.

Die vorliegende Erfindung hat es sich zur Aufgabe gemacht, die gravierenden Nachteile der dem Stand der Technik entsprechenden parasitiziden Desinfektionsmittel zu überwinden.

Dabei war die Verwendung von Substanzen, die eine mittelbare oder unmittelbare Gefährdung der Umwelt darstellen, zu vermeiden.

Diese Aufgabe wird für ein Desinfektionsmittel zur Bekämpfung von Parasitosen und Abtötung von parasitären, invasiven Dauerformen auf Basis eines Gemisches dadurch gelöst, daß das Gemisch besteht aus
a) einer Kombination aus einem oder mehreren Phenolen und keratolytisch wirksamen organischen Säuren als Desinfektionswirkstoff,
b) Ethylenglykoldialkylether der allgemeinen Formel H₃CO(CH₂-CH₂-O)ₙCH₃ (n=1-8) oder einem Gemisch aus verschiedenen Kettenlängen dieser Ether,
c) Natrium- oder Kaliumsalzen der Alkylsulfonate bzw. - sulfate mit primären oder sekundären Ketten der Länge C₈ - C₁₈ oder ein Gemisch daraus als anionische Tenside.

Überraschend wurde nun gefunden, daß auf die Anwendung der toxischen Lösungsmittel in parasitiziden Desinfektionsmittel verzichtet werden kann, wenn man als Wirkstoff eine Kombination aus Phenolen und solchen organischen Säuren verwendet, die keratolytische Eigenschaften besitzen.

Erfindungsgemäß werden für die Herstellung der Desinfektionsmittel Ethylenglykoldialkylether der allgemeinen Formel H₃CO(CH₂-CH₂-O)ₙCH₃ (n=1-8) als Lösungsmittel verwendet. Zusätzlich können Glykole und/oder Alkohole der Kettenlänge 2 - 4 sowie deren isomere Formen einzeln oder im Gemisch zugegeben werden. Dabei ist es nicht Aufgabe der Lösungsmittel, unmittelbar zur parasitiziden Wirkung -vergleichbar dem Perchlorethylen oder Schwefelkohlenstoff- beizutragen, sondern als notwendige Hilfsstoffe zur Herstellung eines Desinfektionsmittelkonzentrates (Lösung der Phenolderivate und Säuren) zu dienen.

Anionische Tenside vom Typus der n-Alkyl-(C₁₀-C₁₃)-arylsulfonate und/oder Alkylsulfonate bzw. -sulfate als Natrium- oder Kaliumsalze mit prim. oder sek. Ketten der Länge C₈-C₁₈ oder ein Gemisch daraus dienen in den erfindungsgemäßen Desinfektionsmitteln als Emulgatoren für die Wirkstoffkombination sowie zur Verbreitung der Wirkstoffe auf den zu desinfizierenden Flächen. Beispiele für n-Alkyl-(C₁₀-C₁₃)-arylsulfonate sind insbesondere Gemische aus n-Alkyl-(C₁₀-C₁₃)-benzolsulfonaten, deren Alkylrest 10 bis 13 Kohlenstoffatome aufweist. Beispielhafte Alkylreste mit 10 bis 13 Kohlenstoffatomen sind die n-Decyl-, die n-Undecyl-, die n-Dodecyl und die n-Dodecylgruppe. Primäre oder sekundäre Ketten der Länge (C₈-C₁₈) werden durch folgende Gruppen veranschaulicht: n-Octyl-, 2-Octyl-, n-Nonyl-, 2-Nonyl-, n-Decyl-, 2-Decyl-, n-Undecyl-, 2-Undecyl-, n-Dodecyl-, 2-Docecyl-, n-Tridecyl-, 2-Tridecyl, n-Tetradecyl-, 2-Tetradecyl-, n-Pentadecyl-, 2-Pentadecyl-, n-Hexadecyl-, 2-Hexadecyl-, n-Heptadecyl-, 2-Heptadecyl-, n-Octadecyl- und 2-Octadecyl-gruppe.

Die erfindungsgemäßen Desinfektionsmittel sind dadurch gekennzeichnet, daß sie einen oder mehrere phenolische Desinfektionswirkstoffe enthalten. Als solche kommen Phenol, substituierte Phenole, Kresole und halogenierte Kresole, insbesondere 2-Methyl-phenol, 3-Methylphenol, 4-Methylphenol, 4-Äthyl-phenol, 2,4-Dimetnyl-phenol, 2,5-Dimethyl-phenol, 3,4-Dimethyl-phenol, 2,6-Dimethyl-phenol, 4-n-Propyl-phenol, 4-n-Butylphenol, 4-n-Amyl-phenol, 4-n-Hexyl-phenol, Thymol, o-Cyclohexyl-p-chlor-phenol, o-n-Amyl-p-chlor-phenol, o-n-Hexyl-p-chlor-phenol, p-Chlor-m-kresol, 4-tert. Butyl-2,6-dichlor-phenol, 6-tert.-Butyl-4-chlor-m-kresol, 4-Äthyl-4-chlor-phenol, 4-chlor-3,5-xylenol, 2,4-Dichlor-3,5-xylenol, p-Phenyl-phenol, o-Phenyl-phenol, 2-Benzyl-phenol, p-Chlor-o-phenyl-phenol, Benzyl-4-chlor-m-kresol und 4-Chlorbenzyl-dochlor-m-kresol in Betracht, jedoch vorzugsweise p-Chlor-m-kresol. Als organische Säuren mit keratolytischen Eigenschaften werden Salicylsäure, Thioglykolsäure und Ameisensäure oder ein Gemisch aus diesen Säuren verwendet.

Die Mischungen sind so zusammengesetzt, daß das Gewichtsverhältnis der keratolytischen Säuren zum Phenolanteil zwischen 1:9 und 9:1 beträgt und deren Summe zwischen 25 und 50 Gew.% bezogen auf das Gesamtgewicht des Desinfektionsmittelkonzentrats liegen kann.

Die Ethylenglykoldialkylether können mit Alkoholen der Kettenlänge 2-4 und deren isomeren Formen vermischt sein und einzeln oder im Gemisch mit den Alkoholen zwischen 15 und 60% bezogen auf das Gesamtgewicht im Desinfektionsmittelkonzentrat enthalten sein.

Die Gemische aus Ethylenglykoldialkylethern und Alkoholen können zusätzlich Ethylenglykol oder Propylenglykol enthalten, wobei der Anteil der Glykole zwischen 10 und 50 Gew.% des Gesamtlösungsmittelanteils liegen kann.

Die Alkylsulfonate mit n-Alkyl- (C₁₀-C₁₃)-arylsulfonaten und deren Kalium- oder Natriumsalzen können im Gemisch eingesetzt werden und der Gesamtanteil des anionischen Tensides einzeln oder im Gemisch kann 5 bis 30 Gew.% (absolut Tensid) bezogen auf das Gesamtgewicht des Desinfektionsmittelkonzentrats betragen.

Eine weitere Verbesserung des Spreitungsverhaltens des Desinfektionsmittels kann erreicht werden, wenn die anionischen Tenside im Gemisch mit Nonylphenolpolyglykolether (2-18 EO) eingesetzt werden, wobei der Anteil des nichtionogenen Tensids zwischen 0,2 und 2 Gew.% bezogen auf das Gesamtgewicht des Desinfektionsmittelkonzentrats liegen kann.

Die Verwendung keratolytisch wirksamer Säuren und die dadurch erzielte Durchlässigkeit einer chitinhaltigen Parasitenmembran macht das Desinfektionsmittel besonders geeignet zur Bekämpfung von Parasitosen, insbesondere zur Abtötung von Askariden und Kokzidien sowie deren invasionsfähigen Dauerformen.

Als wirkungsvoll erwiesen in der Anwendung haben sich die Applikation des Desinfektionsmittels als wäßrige verdünnte Lösung, die zwischen 0,5 und 10 Gew.% des Desinfektionsmittelkonzentrats enthält.

Die erfindungsgemäßen Desinfektionsmittel erzielen bessere Ergebnisse als sie nach den Prüfungsrichtlinien der DVG (Deutsche Veterinärmedizinische Gesellschaft) für chemische Desinfektionsmittel gefordert werden. Darüber hinaus wurde zur Prüfung der Wirksamkeit gegen Kokzidienoozysten eine neue Test-Methode angewendet, die die DVG - Methode, die praktisch eine qualitative Bewertung der Wirksamkeit darstellt, durch ein quantitatives Verfahren ergänzt. Innerhalb dieses Prüfverfahrens, das de facto eine deutliche Verschärfung der Testbedingungen darstellt (Erläuterung im Anhang), zeigten die erfindungsgemäßen Desinfektionsmittel wiederum ihre Überlegenheit gegenüber den Desinfektionsmitteln, die dem Stand der Technik entsprechen.

Überraschenderweise wurde nämlich gefunden, daß auch die erfindungsgemäßen, keratolytisch wirksamen Säuren die Hülle der Askarideneier durchdringen können. Insbesondere war nicht zu erwarten, daß diese Säuren nicht von der bereits genannten chitinähnlichen Schicht zurückgehalten werden.

Keratolytische Wirksamkeit bedeutet nämlich, daß Keratine zersetzt werden. Keratine sind aber Proteine, im Unterschied zu den chitinähnlichen Substanzen der Askarideneier, di ein Polysaccharid darstellen. Wegen der unterschiedlichen Struktur von Proteinen und von Polysachariden war nicht zu erwarten, daß die keratolytisch wirksamen Säuren im Gemisch mit den erfindungs gemäßen Phenolen die chitinähnliche Schicht durchwandern können, denn Proteine weisen hydrolytisch leicht spaltbare Amidbindungen auf, während Polysacharide, vor allem chitinähnliche Substanzen, wesentlich schwerer hydrolytisch zu zersetzten sind. Aber auch die Überwindung einer Barriere in Form einer Chitinschicht auf eine andere Weise als durch Hydrolyse ist nur schwer zu erreichen. So ist bekanntermaßen der Chitinpanzer von Insekten besonders widerstandsfähig. Es ist daher völlig überraschend, wenn ein keratolytisch wirksame Säuren enthaltendes Gemisch ohne Zusatz der obenerwähnten organischen Lösemittel gegen die sehr resistenten Dauerformen von Parasiten eingesetzt werden kann.

Weitere vorteilhafte Zusammensetzungen des Gemisches sind in den Ansprüchen 2 bis 8 genannt.

Von der deutschen Veterinärmedizinischen Gesellschaft (DVG) werden gemäß Richtlinie für die Prüfung Chemischer Desinfektionsmittel für die Beurteilung der parasitiziden Wirksamkeit Askariden und Askarideneier des Typs Askaris suum verwendet. Zur Erlangung eines Wirksamkeitszertifkates ist die sichere Abtötung beider Arten innerhalb eines bestimmten Zeitraumes vorgeschrieben. Dies gilt ebenso für die Kokzidien und die Abtötung ihrer Dauerformen, wie Kokzidienoozysten vom Typ Eimeria tenella. Die beiden genannten Arten von Kleinstlebewesen, insbesondere ihre Dauerformen (Eier bzw. Oozysten), gelten als besonders schwer abtötbar.

Die nachfolgend gezeigten Beispiele sollen die Erfindung erläutern.

Die in den Beispielen vorkommenden Zahlen hinter den genannten Komponenten bezeichnen die eingesetzten Gewichtsteile der Komponenten.

| | | |
|---|---|---|
| Beispiel 1 | sek.-Alkyl-(C₈-C₁₈)-sulfonat-Na | 10,0 |
| | Ethylenglykoldialkylether | 15,0 |
| | Ethylenglykol | 10,0 |
| | Propanol-2 | 10,0 |
| | 4-Chlor-3-methyl-phenol | 20,0 |
| | Ameisensäure | 5,0 |
| | Thioglykolsäure | 5,0 |
| | Wasser (vollentsalzt) | 25,0 |
| Beispiel 2 | Alkylarysulfonat-Na | 4,2 |
| | Dodecylsulfat-Na | 5,0 |
| | Nonylphenolpolyglykolether | 0,8 |
| | Ethylenglykodialkylether | 35,0 |
| | 4-Chlor-3-methyl-phenol | 25,0 |
| | Salicylsäure | 4,0 |
| | Ameisensäure | 6,0 |
| | Wasser (vollentsalzt) | 20,0 |
| Beispiel 3 | sek.-Alkyl-(C₈-C₁₈)-sulfonat-Na | 6,0 |
| | Dodecylsulfat-Na | 3,0 |
| | Ethylenglyokoldialkylether | 20,0 |
| | Ethylenglykol | 20,0 |
| | 4-Chlor-3-methyl-phenol | 25,0 |
| | Salicylsäure | 8,0 |
| | Wasser (vollentsalzt) | |

Die Ergebnisse der Wirksamkeitsprüfungen mit den vorgestellten Beispielformulierungen an den parasitären Dauerformen Askaris suum und Eimeria tenella gemäß DVG-Methode und im Falle der Kokzidienoozysten zusätzlich mit einer erweiterten Prüfmethode sind im Anhang dargestellt.

Wirksamkeitsprüfung gegen Askaris suum Die nachfolgend beschriebenen Ergebnisse wurden durch Anwendung des Prüfverfahrens der DVG zur Prüfung chemischer Desinfektionsmittel erhalten. Gemäß den Richtlinien müssen 90% der Askarideneier abgetötet sein, um ein Präparat als Wirksam zu deklarieren. Die Wirksamkeitsprüfungen wurden sowohl im Suspensions- als auch Keimträgerversuch vorgenommen.

Die unbehandelten Kontrollen embryonierten zu >95%.

| Desinfektionsmittel nach Beispiel 1) | Anwendungskonzentration: 5% |
|---|---|
| Einwirkungszeit (Min.) | Anzahl embryonierter Wurmeier |
| 2 | 1 |
| 5 | 0 |
| 10 | 0 |
| 20 | 0 |

| Desinfektionsmittel nach Beispiel 2) | Anwendungskonzentration: 5% |
|---|---|
| Einwirkungszeit (Min.) | Anzahl embryonierter Wurmeier |
| 2 | 0 |
| 5 | 0 |
| 10 | 0 |
| 20 | 0 |

| Desinfektionsmittel nach Beispiel 3) | Anwendungskonzentration: 5% |
|---|---|
| Einwirkungszeit (Min.) | Anzahl embryonierter Wurmeier |
| 2 | 0 |
| 5 | 0 |
| 10 | 0 |
| 20 | 0 |

### Prüfung der Wirksamkeit gegen Kokzidienoozysten

Gemäß DVG-Richtlinien müssen Kokzidienoozysten durch ein Desinfektionsmittel innerhalb einer Einwirkungszeit von 5 Minuten derart inaktiviert werden, daß sie im Tierkörper nicht mehr zu Infektionen führen. Den Hühnerküken werde 200.000 desinfizierte Oozysten inokuliert und nach 8 Tagen - rein qualitativ- folgende Parameter erfaßt: Makroskopische Beurteilung der Därme hinsichtlich Kokzidiose-Läsionen und Eimeria tenella- Entwicklungsformen durch Schleimhautabstriche. Als Vergleich und Bezugsgröße zum zu prüfenden Desinfektionsmittel dient eine 6%ige Ammoniumhydroxidlösung mit der eine gleich große Zahl Oozysten behandelt und dann einer Vergleichsgruppe Küken inokuliert wird.

In dieser zuvor beschriebenen Versuchsanordnung erreichten die Desinfektionsmittel der Beispiele 1 bis 3 die erforderliche Bewertung um als wirksam zu gelten.

### Quantitatives Verfahren zur Bestimmung der Wirksamkeit gegen Kokzidienoozysten

Es ist seit langem bekannt, daß selbst Inokulationen mit nur einer Oozyste bei ca. 50% der Tiere zu detektierbaren Infektionen führen. Inokulationen mit zwei Oozysten führen regelmäßig zu Infektionen. Bei einer Inokulationsdosis von 200.000 Oozysten müßte ein 99,999%iger Desinfektionserfolg gemäß Richtlinien als unzureichend eingestuft werden. Aufgrund der Schwankungsbreite der Ergebnisse (mehrere Kükengruppen) im DVG-Test und aufgrund der qualitativen Auswertung ist eine derartig genaue Angabe im Hinblick auf relevante statistische Parameter gar nicht möglich. Daher wurden die erfindungsgemäßen Desinfektionsmittel zusätzlich einem neuerdings zur Verfügung stehenden quantitativen Prüfverfahren unterzogen. Das Verfahren beruht auf der Beobachtung, daß bei Inokulationsdosen ab 20.000, relativ zur Inokulationsdosis, im Tierkörper weniger Oozysten produziert werden als bei Verabreichung geringer Dosen (Crowding Effect). Für Inokulationsdosen von weniger als 2000 Oozysten ergibt sich ein reproduzierbares, regelhaftes lineares Verhältnis zwischen dem Wert des natürlichen Logarithmus der Inokulationsdosis und dem Wert des dekadischen Logarithmus der Oozystenproduktion im Tierkörper
(OpG = Oozysten / g Blinddarminhalt).

Nach der Regressionsanalyse erhält man die Eichfunktion der Geraden und präzise statistische Parameter, die eindeutig Qualität und Aussagewert des jeweiligen Tierversuchs determinieren.

Die Infektiosität eines Inokulums unbekannter Größe läßt sich somit aus der Zahl der produzierten Oozysten (Zahl der Oozysten pro g Blinddarm) vergleichsweise exakt berechnen.

Abweichend von der DVG-Methode wird bei diesem Verfahren für das Desinfektionsmittel keine Wirksamkeit nach 5 Minuten Einwirkungsdauer gefordert, sondern in Anlehnung an die Praxis der Stallinfektion nach 60 Minuten Einwirkungsdauer eine Abtötungsrate von >90% als ausreichend für den Nachweis der Wirksamkeit angesehen. Als Bezugsgröße zum zu prüfenden Desinfektionsmittel dient hier ebenfalls eine 6%ige Ammoniumhydroxidlösung.

Die Wirksamkeit der erfindungsgemäßen Beispiele 1 bis 3 stellt sich bei einer Anwendungskonzentration von 5% in Leitungswasser wie folgt dar:

| | | |
|---|---|---|
| Beispiel 1): | Abtötungsrate: | 96,7 % |
| Beispiel 2): | Abtötungsrate: | 99,4 % |
| Beispiel 3): | Abtötungsrate: | 98,9 % |

Vergleichend dazu wurden handelsübliche, dem Stand der Technik entsprechende Präparate zur Stallinfektion auf Basis Schwefelkohlenstoff, Chloroform und Perchlorethylen getestet. Deren Ergebnisse in der quantitativen Wirksamkeitsprüfung bewegten sich lediglich zwischen Abtötungsraten von 55,2% bis 83,7%, wodurch die große Leistungsfähigkeit der erfindungsgemäßen Desinfektionsmittel demonstriert wird.

In den Graphiken 1 bis 3 werden die Ergebnisse der erfindungsgemäßen Desinfektionsmittel aus der quantitativen Wirksamkeitsprüfung nochmals verdeutlicht.

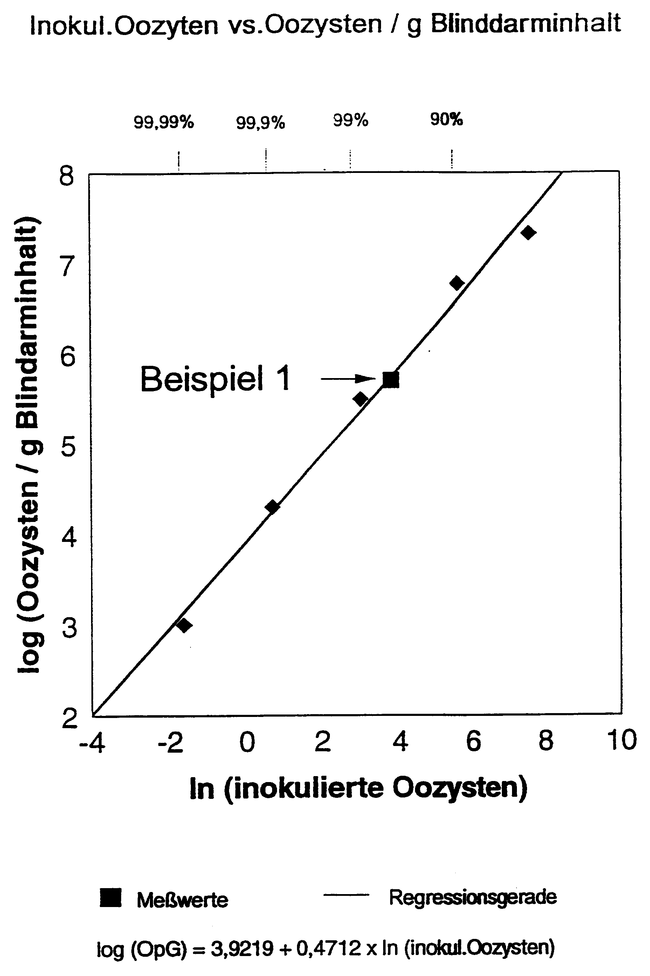

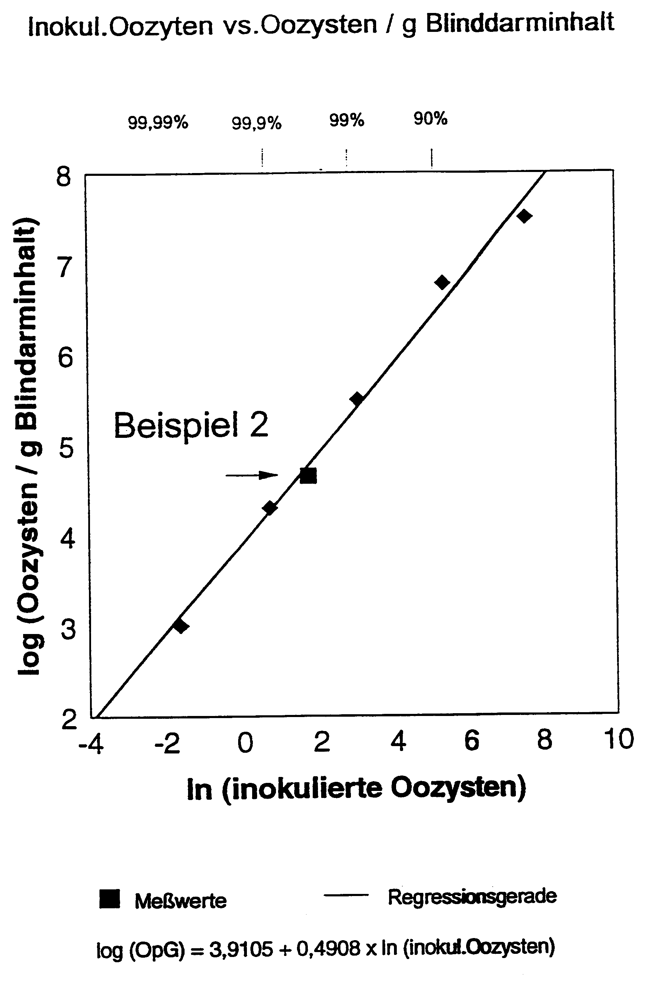

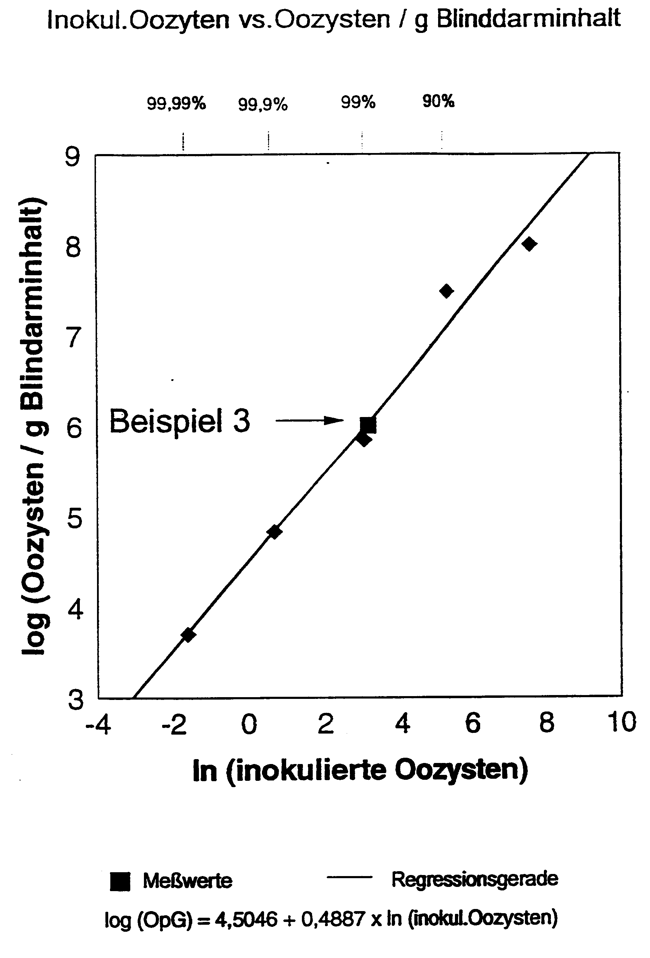

## Patentansprüche

1. Desinfektionsmittel zur Bekämpfung von Parasitosen und Abtötung von parasitären, invasiven Dauerformen auf Basis eines Gemisches, **dadurch gekennzeichnet,** daß das Gemisch besteht aus
a) einer Kombination von einem oder mehreren Phenolen mit keratolytisch wirksamen organischen Sauren als Desinfektionswirkstoff,
b) Ethylenglykoldialkylether der allgemeinen Formel H₃CO(CH₂-CH₂-O)ₙCH₃ (n=1-8) oder einem Gemisch aus verschiedenen Kettenlängen dieser Ether,
c) Natrium- oder Kaliumsalzen der Alkylsulfonate bzw. -sulfate mit primären oder sekundären Ketten der Lage C₈ - C₁₈ oder ein Gemisch daraus als anionische Tenside.

2. Desinfektionsmittel nach Anspruch 1, **dadurch gekennzeichnet**, daß als Phenol 4-Chlor-3-methyl-phenol und als keratolytisch wirksame organische Saure Ameisensäure, Salicylsäure und Thioglykolsaure einzeln oder im Gemisch miteinander verwendet werden.

3. Desinfektionsmittel nach Anspruch 1 oder 2, **dadurch gekennzeichnet,** daß das Gewichtsverhältnis der keratolytischen Sauren zum Phenolanteil zwischen 1:9 und 9:1 beträgt und deren Summe zwischen 25 und 50 Gew.% bezogen auf das Gesamtgewicht des Desinfektionsmittelkonzentrats liegen kann.

4. Desinfektionsmittel nach Anspruch 1 oder 3, **dadurch gekennzeichnet,** daß die Ethylenglykoldialkylether mit Alkoholen der Kettenlänge 2-4 und deren isomeren Formen vermischt sein können und einzeln oder im Gemisch mit den Alkoholen zwischen 15 und 60% bezogen auf das Gesamtgewicht im Desinfektionsmittelkonzentrat enthalten sein können.

5. Desinfektionsmittel nach einem der Ansprüche 1 bis 4, **dadurch gekennzeichnet,** daß Gemische aus Ethylenglykoldialkylethern und Alkoholen zusätzlich Ethylenglykol oder Propylenglykol enthalten können, wobei der Anteil der Glykole zwischen 10 und 50 Gew.% des Gesamtlösungsmittelanteils liegen kann.

6. Desinfektionsmittel nach einem der Ansprüche 1 bis 5, **dadurch gekennzeichnet,** daß die Alkylsulfonate mit n-Alkyl- (C₁₀-C₁₃)-arylsulfonaten und deren Kalium- oder Natriumsalzen im Gemisch eingesetzt werden können und der Gesamtanteil des anionischen Tensides einzeln oder im Gemisch 5 bis 30 Gew.% (absolut Tensid) bezogen auf das Gesamtgewicht des Desinfektionsmittelkonzentrats betragen kann.

7. Desinfektionsmittel nach einem der Ansprüche 1 bis 6, **dadurch gekennzeichnet,** daß die anionischen Tenside im Gemisch mit Nonylphenolpolyglykolether (2-18 EO) eingesetzt werden können, wobei der Anteil des nichtionogenen Tensids zwischen 0,2 und 2 Gew.% bezogen auf das Gesamtgewicht des Desinfektionsmittelkonzentrats liegen kann.

8. Verwendung der Desinfektionsmittel nach einem der Ansprüche 1 bis 7 zur Bekämpfung von Parasitosen, insbesondere zur Abtötung von Askariden und Kokzidien sowie deren invasionsfähigen Dauerformen.

9. Verwendung der Desinfektionsmittel nach einem der Ansprüche 1 bis 7 in wäßrigen verdünnten Lösungen, die zwischen 0,5 und 10 Gew.% des Desinfektionsmittelkonzentrats enthalten können.

## Claims

1. A disinfectant for combatting parasitic infection and destroying parasitical, invasive, dormant microorganisms on the basis of a mixture characterised in that the mixture consists of
a) a combination of one or more phenols and keratolytically effective organic acids as a disinfection active ingredient,
b) ethylene glycol dialkylether of the general formula H₃CO(CH₂-CH₂-O)ₙCH₃ (n=1-8) or a mixture from various chain lengths of this ether,
c) sodium or potassium salts of the alkylsulphonates or alkylsulphates with primary or secondary chains of the length C₈-C₁₈ or a mixture therefrom as an anionic tenside.

2. A disinfectant according to claim 1, characterised in that as phenol, 4-chloro-3-methyl-phenol, and as keratolytically effective organic acids formic acid, salicyl acid and thioglycol acid are used individually or in mixture with one another.

3. A disinfectant according to claim 1 or 2, characterised in that the weight ratio of the keratolytic acids to the phenol component is between 1:9 and 9:1 and their sum may lie between 25 and 50% by weight with respect to the total weight of the disinfectant concentrate.

4. A disinfectant according to claim 1 or 3, characterised in that the ethylene glycol dialkylethers may be mixed with alcohols of the chain length 2-4 and their isomer forms, and individually or in mixture with the alcohols may be contained in the disinfectant concentrate between 15 and 60% with respect to the total weight.

5. A disinfectant according to one of claims 1 to 4, characterised in that the mixtures of ethylene glycol dialkylethers and alcohols may additionally contain ethylene glycol or propylene glycol, wherein the component of the glycols may lie between 10 and 50% by weight of the total solvent component.

6. A disinfectant according to one of claims 1 to 5, characterised in that the alkylsulphonates with n-alkyl-(C₁₀-C₁₃)-arylsulphonates and their potassium or sodium salts may be applied in mixture and the total component of the anionic tensides individually or in the mixture may be 5 to 10% by weight (absolute tenside) with respect to the total weight of the disinfectant concentrate.

7. A disinfectant according to one of claims 1 to 6, characterised in that the anionic tensides may be applied in mixture with nonylphenolpolyglycolether (2-18 EO), wherein the component of the non-ionogenic tenside may lie between 0.2 and 2% by weight with respect to the total weight of the disinfectant concentrate.

8. The use of a disinfectant according to one of claims 1 to 7, for combatting parasitic infections, in particular for destroying ascarides and coccidia as well as their dormant microorganisms capable of invasion.

9. The use of the disinfectant according to one of claims 1 to 7 in aqueous, diluted solutions, which may contain between 0.5 and 10% by weight of the disinfectant concentrate.

## Revendications

1. Agent désinfectant pour combattre les parasitoses et détruire les formes parasitaires durables invasives à base d'un mélange,
caractérisé en ce que le mélange est constitué de
a) une combinaison d'un ou plusieurs phénols et acides organiques à action kératolytique comme matière active désinfectante,
b) des dialkyléthers d'éthylène glycol de formule générale H₃CO(CH₂-CH₂-O)ₙCH₃ (n=1-8) ou un mélange de diverses longueurs de chaînes de ces éthers,
c) des sels de sodium ou de potassium des alkylsulfonates ou -sulfates avec des chaînes primaires ou secondaires d'une longueur de C₈ à C₁₈ ou un de leurs mélanges comme agents tensioactifs anioniques.

2. Agent désinfectant selon la revendication 1,
caractérisé en ce qu'
on utilise comme phénol le 4-chloro-3-méthyl-phénol et comme acide organique à action kératolytique, l'acide formique, l'acide salicylique et l'acide thioglycolique, isolément ou mélangés.

3. Agent désinfectant selon la revendication 1 ou 2,
caractérisé en ce que
- le rapport pondéral des acides kératolytiques à la fraction phénol s'établit entre 1:9 et 9:1 et
- leur somme peut se situer entre 25 et 50 % en poids par rapport au poids total du concentré d'agent désinfectant.

4. Agent désinfectant selon la revendication 1 ou 3,
caractérisé en ce que
les dialkyléthers d'éthylène glycol peuvent être mélangés avec des alcools d'une longueur de chaîne de 2 à 4 et leurs formes isomères peuvent être isolées ou contenus dans des mélanges avec des alcools à raison de 15 à 60 % par rapport au poids total dans le concentré d'agent désinfectant.

5. Agent désinfectant selon l'une des revendications 1 à 4,
caractérisé en ce que
les mélanges de dialkyléthers d'éthylène glycol et d'alcools peuvent contenir en outre de l'éthylène glycol ou du propylène glycol, la proportion des glycols pouvant se situer entre 10 et 50 % en poids de la proportion totale de solvant.

6. Agent désinfectant selon l'une des revendications 1 à 5,
caractérisé en ce que
- les alkylsulfonates peuvent être utilisés avec les n-alkyle en C₁₀ à C₁₃-arylsulfonates et leurs sels de potassium ou de sodium mélangés, et
- la proportion totale de l'agent tensioactif anionique isolé ou en mélange peut s'élever à 5 à 30 % en poids (agent tensioactif absolu) par rapport au poids total du concentré d'agent désinfectant.

7. Agent désinfectant selon l'une des revendications 1 à 6,
caractérisé en ce que
les agents tensioactifs anioniques peuvent être utilisés en mélange avec des polyglycoléthers de nonylphénol (2-18 OE), la proportion de l'agent tensioactif non ionogène pouvant se situer entre 0,2 et 2 % par rapport au poids total du concentré d'agent désinfectant.

8. Utilisation de l'agent désinfectant selon l'une des revendications 1 à 7 pour combattre les parasitoses, en particulier pour détruire les ascarides et les coccidies ainsi que leurs formes durables invasives.

9. Utilisation des agents désinfectants selon l'une des revendications 1 à 7 dans des solutions aqueuses diluées, qui peuvent contenir entre 0,5 et 10 % en poids de concentré d'agent désinfectant.
